# EUROPEAN PATENT APPLICATION

(11) **EP 3 967 693 A1**
(43) Date of publication of application: **16.03.2022**
(21) Application number: 20802939.7
(22) Date of filing: 08.05.2020
(51) Int. Cl.: C07D 471/04, A61K 31/519, A61P 35/00

(54) **AMORPHOUS PI3K INHIBITOR AND PHARMACEUTICAL COMPOSITION COMPRISING SAME**

(30) Priority: 09.05.2019 KR 20190054507
(71) Applicant: Boryung Pharmaceutical Co., Ltd., Seoul 03127 (KR)
(72) Inventor: KIM, Seong Heon, Seoul 07981 (KR); LEE, Joon Kwang, Gwangmyeong-si Gyeonggi-do 14293 (KR); SUN, Yong Ho, Gunpo-si Gyeonggi-do 15804 (KR); KIM, Ji Han, Seoul 07444 (KR)
(74) Representative: HGF
(86) International application number: PCT/IB2020/054365
(87) International publication number: WO 2020/225782

(57) **Abstract**

The present invention relates to an amorphous PI3K inhibitor and a pharmaceutical composition comprising same. According to the present invention, a novel amorphous PI3K inhibitor having improved solubility and bioavailability and excellent stability and a pharmaceutical composition comprising same can be provided.

## Description

### [Technical Field]

The present invention relates to an amorphous PI3K inhibitor and a pharmaceutical composition including the same.

### [Background Art]

Phosphatidylinositol 3-kinase (PI3 kinase; PI3K) is a lipid kinase, which phosphorylates lipid molecules instead of proteins, and plays an important role in cell survival, signal transduction, control of membrane trafficking, etc. If a problem occurs to such control, a cancer, inflammatory disease, autoimmune disease, etc. occurs.

Recently, study results for developing a compound having a novel structure effective in selectively inhibiting PI3 kinase have been reported, and specifically International Publication No. WO 2016/204429 discloses a compound which has PI3 kinase inhibitory activity and is useful for the treatment of cancer, autoimmune diseases, respiratory diseases and the like, which is incorporated herein by reference.

When designing a suitable dosage form in the process of preparing a drug, the physical state of raw materials of the drug, that is, whether the state is crystalline or amorphous, is very important.

However, an amorphous form generally has difficulty in filtering due to relatively small particles and a wide surface area and has many problems in that a shelf life becomes short due to instability and it is difficult to release a drug and adjust a blood concentration.

### [Prior Art References]

### [Patent Documents]

(Patent Document 1) International Publication No. WO 2016/204429

### [Disclosure of Invention]

### [Technical Problem]

An object of the present invention is to provide an amorphous PI3K inhibitor represented by formula I below capable of enhancing solubility and bioavailability with excellent stress stability and long-term stability.

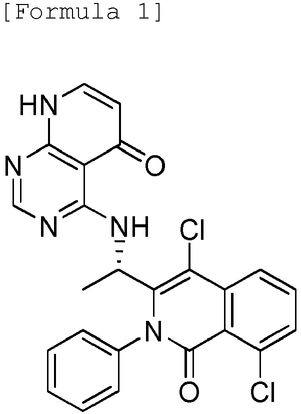

An object of the present invention is to provide a pharmaceutical composition including an amorphous form of a compound represented by formula I as an effective ingredient.

### [Technical Solution]

The present inventors have made efforts to find an long-term stable and industrially applicable amorphous form of (S)-4-((1-(4,8-dichloro-1-oxo-2-phenyl-1,2-dihydroisoquinoline-3-yl)ethyl)amino)pyrido[2,3-d]pyrimidin-5(8H)-one (hereinafter, the compound of formula 1), which is one of heteroaryl derivatives known to have P13 kinase inhibitory activity, so that this form may be used as a pharmaceutical preparation. In result, it is shown that by converting the compound into an amorphous form, the amorphous form is stably maintained without a change in content in the long term due to excellent stability depending on accelerated, long-term and stress storage conditions, excellent thermal stability and excellent stability according to pH, and an amorphous P13K inhibitor may show high solubility, thus, the amorphous form exhibits an excellent pharmacological effect with a high concentration in blood and high bioavailability. Thus, since the amorphous form of the compound shows high bioavailability when orally administered and thus may exhibit an excellent therapeutic effect even with a small amount of use, the present inventors have confirmed that the amorphous form may be safe, have less side effects, and remarkably improve the convenience of taking medication for patients, thereby completing the present invention.

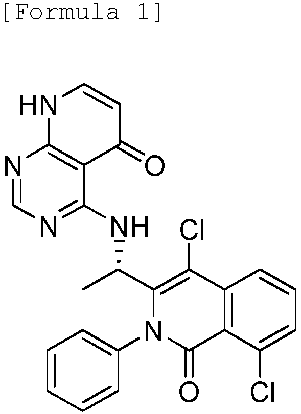

The present invention provides an amorphous P13K inhibitor.

The amorphous P13K inhibitor of the present invention may have high bioavailability and thus provide an excellent therapeutic effect and remarkably high stability at the same time.

The amorphous P13K inhibitor of the present invention may have excellent stability according to accelerated, long-term and stress storage conditions, excellent thermal stability, and excellent stability according to pH, and thus may be stably maintained without any change in content in the long term. Thus, the same state may be maintained even after producing a preparation by using the amorphous P13K inhibitor of the present invention or producing a preparation containing the same, thus the content uniformity of the preparation may be stably maintained for a long period of time, and excellent stability may be maintained for a long period of time due to remarkably less occurrence of impurities. In addition, the amorphous P13K inhibitor may be easily applied to mass production.

The amorphous P13K inhibitor of the present invention may exhibit high solubility and thus a high concentration in blood, thereby exhibiting an excellent pharmacological effect with high bioavailability. Thus, the amorphous PI3K inhibitor of the present invention may be effectively used as a novel effectiveingredient in a pharmaceutical composition capable of effectively treating cancer diseases.

The amorphous P13K inhibitor of the present invention may show high bioavailability when orally administered, and thus may exhibit an excellent therapeutic effect even with a small amount of use, so that the inhibitor may be safe, have fewer side effects, and remarkably improve the convenience of taking medication for patients.

Hereinafter, the present invention will be described in more detail.

### Amorphous P13K inhibitor

There is provided an amorphous form of the compound of formula 1 below.

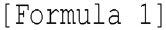

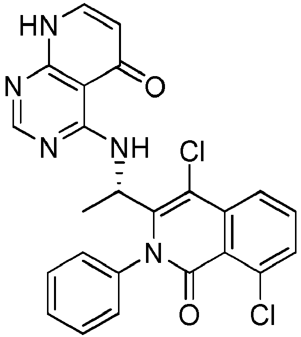

The amorphous PI3K inhibitor of the present invention may have excellent stability according to accelerated, long-term and stress storage conditions, excellent thermal stability, and excellent stability according to pH, and thus may be stably maintained without any change in content in the long term. Thus, the amorphous PI3K inhibitor may have high bioavailability, thereby exhibiting an excellent therapeutic effect while showing high stability.

In general, in spite of high solubility and thus high bioavailability, the amorphous form may have low stability and thus easily generate impurities, causing stability problems. However, the amorphous PI3K inhibitor of the present invention may have excellent stability according to accelerated, long-term and stress storage conditions, excellent thermal stability, and excellent stability according to pH, and thus may be stably maintained without any change in the content in the long term. Thus, the amorphous PI3K inhibitor may have high bioavailability, thereby exhibiting an excellent therapeutic effect while showing high stability.

The amorphous P13K inhibitor of the present invention may have high purity, may be safe, and may have excellent stability to heat, pH and humidity, and thus may be stably maintained without any change in content in the long term. Thus, the same state may be maintained even after producing a preparation by using the amorphous P13K inhibitor of the present invention or producing a preparation containing the same, and thus the content uniformity of the preparation may be stably maintained for a long period of time and may be easily applied to mass production. In addition, the amorphous P13K inhibitor of the present invention may exhibit high solubility and thus a high concentration in blood, thereby exhibiting an excellent pharmacological effect with high bioavailability. Thus, the amorphous PI3K inhibitor of the present invention may be effectively used as a novel effective ingredient in a pharmaceutical composition capable of treating cancer diseases. Accordingly, since the amorphous P13K inhibitor of the present invention shows high bioavailability when orally administered and thus may exhibit an excellent therapeutic effect even with a small amount of use, the inhibitor may be safe, have fewer side effects, and remarkably improve the convenience of taking medication for patients.

In the specification, the term "amorphous" means a state in which compound molecules are aggregated without forming a specific crystal form, and also means to include even a mixture in which substantially no crystal form is observed in the X-ray powder diffraction pattern, even if a trace amount of crystalline molecules is included.

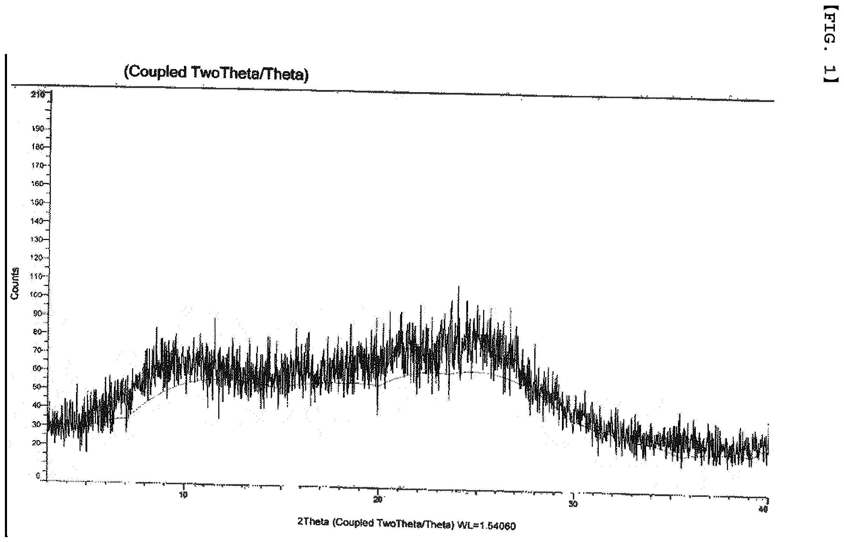

FIG. 1 is a view showing the results of X-ray powder diffraction (PXRD) analysis pattern of the amorphous compound according to the present invention. In one embodiment of the present invention, the amorphous compound may have an X-ray powder diffraction peak spectrum as shown in [FIG. 1].

As confirmed in FIG. 1, the amorphous compound of the present invention did not have a peak appearing at a specific value in an X-ray powder diffraction pattern, and showed an X-ray powder diffraction analysis pattern of diffused halo, which is typical of an amorphous material. Accordingly, it was confirmed that the product is amorphous without crystallinity.

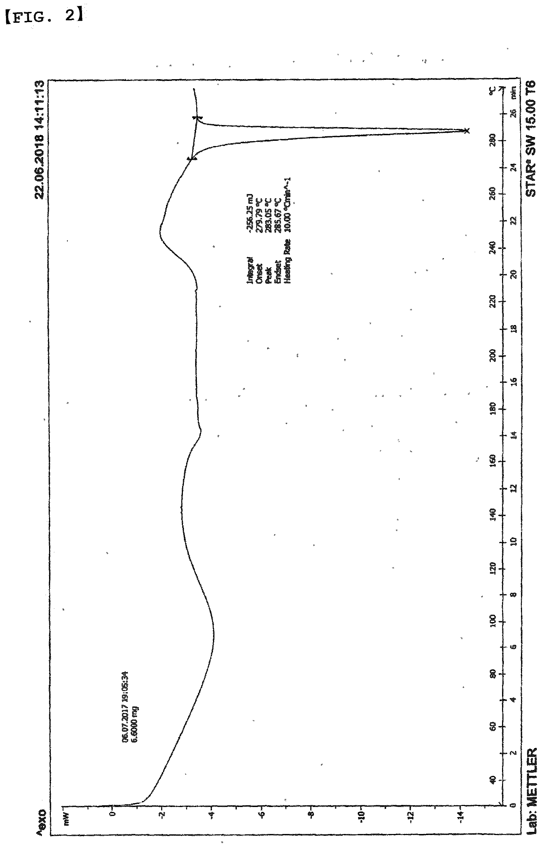

In one embodiment of the present invention, the amorphous compound may have a differential scanning calorimetry (DSC) endothermic transition at 281 to 285°C when a heating rate is 10°C/min, and specifically may have a DSC spectrum as shown in [FIG. 2], but is not limited thereto.

As confirmed in FIG. 2, the amorphous compound of the present invention may exhibit a sharp endothermic peak at 281 to 285°C.

### Pharmaceutical composition including amorphous compound as effective ingredient

The present invention provides a pharmaceutical composition including the amorphous compound of above formula 1 as an effective ingredient.

In the present specification, the term "effective ingredient" means a material or a group of materials (including a herb medicine, etc., of which a pharmacologically effective ingredient, etc., has not been identified yet) expected to directly or indirectly express the efficacy and effect of a composition thereof through an intrinsic pharmacological action, including a main ingredient.

The pharmaceutical composition may be used for preventing or treating cancer, but is not limited thereto.

In the present invention, the term "cancer" means all the conditions, in which a problem medically occurs to regulatory functions of cells for normal division, differentiation or death, then the cells are abnormally subjected to an excessive proliferation to infiltrate into surrounding tissues or organs, and then a mass of cells is formed to destroy or transform an existing structure. Cancer is roughly classified into primary cancer present in a site of origin and metastatic cancer, which spreads from the site of origin to other parts of the human body.

In one embodiment of the present invention, the cancer disease may be any one of lung cancer, non-small cell lung cancer (NSCL), bronchiolo-alveolar cell lung cancer, gastric cancer, gastrointestinal cancer, liver cancer, bone cancer, pancreatic cancer, skin cancer, head and neck cancer, skin or ocular melanoma, uterine cancer, ovarian cancer, rectal cancer, colorectal cancer, colon cancer, breast cancer, sarcoma of uterus, fallopian tube carcinoma, internal endometrium carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penis cancer, prostate cancer, multiple myeloma, chronic or acute leukemia, solid tumor of childhood, lymphoma, bladder cancer, renal cancer, renal cell carcinoma, renal pelvic carcinoma, spinal axis tumor, brainstem glioma or pituitary gland adenoma, but is not limited thereto.

The pharmaceutical composition of the present invention exhibit a remarkably improved preventive and therapeutic activity for cancer by containing the amorphous compound of above formula 1.

In the present invention, the term "prevention" means inhibition or delay of occurrence of disease, disorder or condition. If the occurrence of disease, disorder or condition is inhibited or delayed for an expected period of time, the prevention may be considered as complete.

In the present invention, the term "treatment" means one which partially or completely reduces, ameliorates, alleviates, inhibits or delays a specific disease, disorder, and/or condition or symptom thereof, reduces severity thereof, or reduces the occurrence of at least one symptom or property thereof.

In one embodiment of the present invention, the pharmaceutical composition of the present invention may be formulated into a preparation by using a pharmaceutically acceptable carrier according to a method easily practicable by those skilled in the art, to which the present invention pertains, such that the composition may be prepared in a unit dose form or prepared by being inserted into a multi-dose container.

In the present invention, the term "carrier" means a compound that facilitates the addition of a compound into a cell or tissue, and the term "pharmaceutically acceptable" refer to a composition which is physiologically acceptable and does not conventionally cause an allergic reaction such as gastrointestinal disturbance and dizziness, or other reactions similar thereto, when being administered into humans.

The pharmaceutically acceptable carrier may be one conventionally used in formulating a preparation, including, but not limited thereto, lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, propylhydroxybenzoate, talc, magnesium stearate, mineral oil and the like.

The pharmaceutical composition of the present invention may further include additives such as fillers, anti-coagulants, lubricants, humectants, fragrance, emulsifiers, preservatives, etc. in addition to the above ingredients. In the present invention, a content of additives contained in the pharmaceutical composition is not particularly limited, and may be appropriately adjusted within a range of contents conventionally used in formulating a preparation.

The pharmaceutical composition may be prepared in the form of oral or parenteral preparation apparent to those skilled in the art.

The pharmaceutical composition of the present invention may be intended for oral administration. In the present invention, the term "oral administration" means one, in which a substance prepared for allowing an active substance to be digested is administered into the gastrointestinal tract for absorption. A non-limiting example of the preparation for oral administration may include tablets, troches, lozenges, water soluble suspensions, oil suspensions, prepared powders, granules, emulsions, hard capsules, soft capsules, syrups, elixirs or the like. To formulate the pharmaceutical composition of the present invention into preparations for oral administration, the followings may be used: binders such as lactose, saccharose, sorbitol, mannitol, starch, amylopectin, cellulose, gelatin or the like; excipients such as dicalcium phosphate, etc.; disintegrants such as maize starch, sweet potato starch or the like; lubricants such as magnesium stearate, calcium stearate, sodium stearyl fumarate, polyethylene glycol wax, or the like; etc., in which sweetening agents, flavoring agents, syrups, etc. may also be used. Furthermore, in case of the capsules, liquid carriers such as fatty oil, etc. may be further used in addition to the above-mentioned materials.

A preferable dosage of the pharmaceutical composition of the present invention may vary in a range thereof depending on a patient's state, weight, age, gender, health condition, dietary and constitutional specificity, property of a preparation, a degree of disease, an administration time of the composition, an administration method, an administration period or interval, an excretion rate and drug form, but may be appropriately selected by those skilled in the art. For example, the dosage may be in a range of about 0.1 to 1,000 mg/kg, in a range of about 1 to 800 mg/kg, in a range of about 5 to 600 mg/kg, or in a range of about 10 to 400 mg/kg, and may be preferably in a range of about 50 to 500 mg/kg, but not limited thereto, and may be administered once a month.

In the present specification, the term "effective dosage of a pharmaceutical composition" means an amount of the composition containing an active ingredient sufficient to treat a specific symptom. The dosage may vary depending on a method for formulating the pharmaceutical composition, an administration mode, an administration time and/or an administration route, etc., and may be diversified according to various factors including a type and degree of reaction to be achieved by administration of the pharmaceutical composition, a type of an individual for administration, the individual's age, weight, general health condition, disease symptom or severity, gender, diet and excretion, ingredients of other drug compositions to be used for the corresponding individual at the same time or different times, etc., as well as other similar factors well known in a pharmaceutical field, and those skilled in the art may easily determine and prescribe an effective dosage for intended treatment.

The pharmaceutical composition of the present invention may be administered once a day or several times a day by dividing the daily dosage of the composition. The pharmaceutical composition of the present invention may be administered as an individual therapeutic agent or in combination with other therapeutic agents, and may be administered sequentially or simultaneously with a conventional therapeutic agent. Considering all the above factors, the pharmaceutical composition of the present invention may be administered in such an amount that a maximum effect may be achieved by a minimum amount without a side effect, and such amount may be easily determined by those skilled in the art to which the present invention pertains.

The pharmaceutical composition of the present invention may be intended for oral administration. In the present specification, the term "parenteral administration" means a method of administering subcutaneously, intramuscularly, intravenously, or intraperitoneally using a tube, except for administering orally. A preparation for parenteral administration may be used by being formulated into a dosage form of injectable preparation and external preparation, which are sterilized according to conventional methods respectively, such as aqueous solution, liquid, non-aqueous solvent, suspending agent, emulsion, eye drop, eye ointment, syrup, suppository, aerosol, etc. Preferably, it may be used by preparing a pharmaceutical composition of cream, gel, patch, spray, ointment, plaster, lotion, liniment, eye ointment, eye drop, paste or cataplasma, but is not limited thereto. A composition for local administration may be an anhydrous or aqueous form depending on a clinical prescription. As the non-aqueous solvent and the suspending agent, propylene glycol, polyethylene glycol, vegetable oil like olive oil, injectable ester like ethyl oleate, etc. may be used. A base of the suppository used herein may be witepsol, macrogol, tween 61, cacao butter, laurinum, glycerogelatin, etc.

The pharmaceutically acceptable additive according to the present invention may be contained in an amount of 0.1 to 99.9 parts by weight based on the total weight of the composition.

The pharmaceutical composition of the present invention may contain at least one effective ingredient which shows the same function as or a similar function thereto in addition to the amorphous compound of above formula 1.

### Method for preventing or treating cancer diseases

Other object of the present invention is to provide a method for preventing or treating cancer, the method including administering a pharmaceutical composition for preventing or treating cancer diseases, including the amorphous compound of above formula 1, into an individual in need.

In the present invention, the term "subject" may mean mammals such as monkey, cow, horse, dog, cat, rabbit, rat, mouse, etc., and in particular may include humans. The preventive or therapeutic method may include administering a therapeutically effective amount.

In the present specification, the term "therapeutically effective amount" may refer to an amount of the amorphous compound of above formula 1 of the present invention, which is effective in preventing or treating cancer.

The preventive or therapeutic method of the present invention may include not only dealing with the diseases themselves before expression of their symptoms, but also inhibiting or avoiding such symptoms by administering the pharmaceutical composition for preventing or treating cancer diseases, containing the amorphous compound of above formula 1. In managing the disease, a preventive or therapeutic dose of a certain active ingredient may vary depending on a nature and severity of the disease or condition and a route of administering the active component. A dose and a frequency thereof may vary depending on an individual patient's age, weight and reactions. A suitable dose and usage may be easily selected by those skilled in the art, naturally considering such factors. In addition, the preventive or therapeutic method of the present invention may further include administering a therapeutically effective amount of an additional active agent, which is helpful in treating diseases, along with the pharmaceutical composition containing the amorphous compound of above formula 1 for preventing or treating cancer diseases, in which the additional active agent may achieve a synergy effect or an additive effect together with the pharmaceutical composition for preventing or treating cancer diseases, containing the amorphous compound of above formula 1.

### Use in the manufacture of a medicament for preventing or treating cancer

An object of the present invention is to provide a use of a pharmaceutical composition including an amorphous compound of above formula 1 in the manufacture of a medicament for preventing or treating cancer.

In one embodiment of the present invention, the pharmaceutical composition containing the amorphous compound of above formula 1 of the present invention in the manufacture of a medicament may be mixed with an acceptable carrier, etc., and further contain other agents.

Matters mentioned in the amorphous compound, the pharmaceutical composition, the treatment method and the use of the present invention may be applied the same, if not contradictory to each other.

### [Advantageous Effects]

A novel amorphous compound of the present invention may have excellent stability according to accelerated, long-term and stress storage conditions, excellent thermal stability, and excellent stability according to pH, and thus may be stably maintained without any change in the content in the long term, and the novel amorphous compound may exhibit high solubility and thus a high concentration in blood, thereby showing an excellent pharmacological effect.

The novel amorphous compound of the present invention may show high bioavailability when orally administered, and thus may exhibit an excellent therapeutic effect even with a small amount of use, so that the compound may be safe, have less side effects, and remarkably improve the convenience of taking medication for patients.

### [Brief Description Of The Drawings]

FIG. 1 is a view showing the results of X-ray powder diffraction (PXRD) analysis of the amorphous compound according to Example 1 of the present invention (the unit of 2Theta on the horizontal axis is °) .
FIG. 2 is a view showing the results of a differential scanning calorimetry (DSC) thermogram of the amorphous compound according to Example 1 of the present invention.

### [Mode for Invention]

Hereinafter, the present invention will be described in detail through Examples for better understanding of the present invention. However, the following Examples are provided only for the purpose of illustrating the present invention, and thus the scope of the present invention is not limited thereto. The Examples of the present invention are provided to more completely describe the present invention to those having ordinary skill in the art.

The reagents and solvents mentioned below were purchased from Sigma-Aldrich Korea, TCI, Alfa Aesar unless otherwise specified, and 1100Series of Agilent Technologies was used for the HLPC. ¹H NMR data were measured by using a Bruker Avance III (400 MHz).

In addition, various crystals of (S)-4-((1-(4,8-dichloro-1-oxo-2-phenyl-1,2-dihydroisoquinoline-3-yl)ethyl)amino)pyrido[2,3-d]pyrimidin-5(8H)-one prepared by Examples below were confirmed with an X-ray diffraction pattern by X-ray diffraction and a differential scanning calorimetry (DSC) pattern by a DSC method.

The X-ray diffraction analysis used in the following examples was performed by using a BRUKER AXS X-ray powder diffractometer model D2 PHASER equipped with a rotation measuring instrument and a solid state detector, and measurement was made by a known method in the art using Cu-Kα rays in an analysis range of 0° to 40° at an irradiation rate of 3° per minute. In addition, the DSC measurement performed by the differential scanning calorimetry (DSC) method shows a value measured at 30-300°C with HP DSC1 of METTLER TOREDO while raising a temperature at a rate of 10°C/min.

### Preparation Example 1. Preparation of (S)-4-((1-(4,8-dichloro-1-oxo-2-phenyl-1,2-dihydroisoquinoline-3-yl)ethyl)amino)pyrido[2,3-d]pyrimidin-5(8H)-one

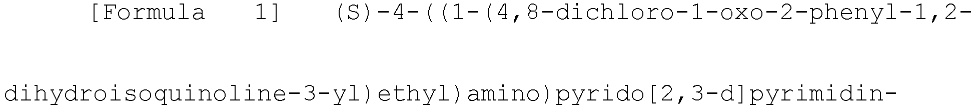

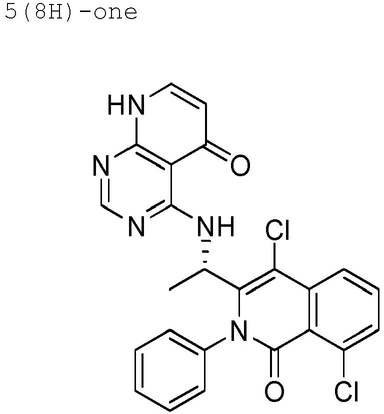

The title compound of (S)-4-((1-(4,8-dichloro-1-oxo-2-phenyl-1,2-dihydroisoquinoline-3-yl) ethyl)amino)pyrido[2,3-d]pyrimidin-5(8H)-one was prepared by the same method as described in Example 10 of the International Publication WO2016/204429.

### Example 1. Preparation of amorphous compound

The 3 g of (S)-4-((1-(4,8-dichloro-1-oxo-2-phenyl-1,2-dihydroisoquinoline-3-yl)ethyl)amino)pyrido[2,3-d]pyrimidin-5(8H)-one prepared in Preparation Example 1 was added to 10 mL of dimethyl sulfoxide (DMSO) and dissolved, after which 100 mL of purified water (H₂O) was added dropwise, slowly cooled down to room temperature, and stirred overnight. The resulting solid was filtered, washed with 15 mL of purified water (H₂O), and dried in a hot air dryer at 45°C, so as to obtain 2.99 g of a solid compound (yield of 100%).

With respect to the solid compound, an X-ray powder diffraction (PXRD) peak and a differential scanning calorimetry (DSC) endothermic peak were analyzed and the results thereof are shown in FIGs. 1 and 2, respectively.

As confirmed in FIG. 1, the solid compound did not have a peak appearing at a specific value, and showed an X-ray powder diffraction analysis pattern of diffused halo, which is typical of an amorphous material. It was confirmed that the product is amorphous without crystallinity.

In addition, as confirmed in above FIG. 2, it could be seen that the solid compound exhibits a sharp endothermic peak at 281 to 285°C.

### Experimental Example 1. Accelerated stability test

The powder of the solid compound prepared in above Example 1 was doubly sealed and packed with an LDPE bag, then sealed and packed with an aluminum bag, and left alone at 40±2°C and RH 75±5% for zero and six months, after which moisture was measured by using Karl Fischer method and the content was measured by using the HPLC method using a standard product, and thus the amount of impurities was measured by using a high speed liquid chromatography (HPLC) under the following conditions. The results thereof are shown in table 1 below.

### <HPLC conditions>

- Detector: Ultraviolet absorption spectrophotometer (wavelength of 307 nm)
- Column: Kromasil 100-5C18 (4.6 × 250 mm, 5*µ*m) or a column similar thereto
- Column temperature: 25°C
- Injection amount: 10 µl
- Mobile phase: Mobile phase A - 20 mmol/L of ammonium acetate buffer solution (pH 4.5, acetic acid), Mobile phase B - acetonitrile:methanol=8:2

| | Mobile phase A (%) | Mobile phase A (%) | Flow rate (mL/min) |
|---|---|---|---|
| 0 | 75 | 25 | 1.0 |
| 5 | 75 | 25 | 1.0 |
| 35 | 30 | 70 | 1.0 |
| 50 | 30 | 70 | 1.0 |
| 51 | 75 | 25 | 1.0 |
| 60 | 75 | 25 | 1.0 |

**[Table 1]**

| Test item | Example 1 | |
|---|---|---|
| | Zero month (Initial) | Six months |
| Individual impurity (%) | 0.04 | 0.04 |
| Total impurity (%) | 0.08 | 0.09 |
| Content (%) | 100.2 | 99.7 |
| Crystal form | Amorphous | Amorphous |

| | | |
|---|---|---|
| (Unit: wt%) | | |

From the results of above table 1, neither a significant change nor a change over time in impurities and contents was observed in the amorphous compound.

Thus, it could be seen that the amorphous compound of Example 1 may stably maintain high purity for six months under accelerated conditions so as to provide excellent stability and a remarkably less occurrence of impurities, thereby maintaining excellent safety for a long period of time with almost no change in moisture content and with almost no absorption of moisture during storage.

### Experimental Example 2. Long-term stability test

The powder of the solid compound prepared in above Example 1 was doubly sealed and packed with an LDPE bag, and sealed and packed with an aluminum bag to carry out a long-term stability test (25±2°C and 60±5% RH) and measure the content of moisture and the amount of impurities by the same method as in Experimental Example 1, and thus the results thereof are shown in table 2 below.

**[Table 2]**

| Test item | Example 1 | |
|---|---|---|
| | Zero month (Initial) | Six months |
| Individual impurity ( % ) | 0.04 | 0.04 |
| Total impurity (%) | 0.08 | 0.09 |
| Content (%) | 100.2 | 100.0 |
| Crystal form | Amorphous | Amorphous |

| | | |
|---|---|---|
| (Unit: wt%) | | |

From the results of above table 2, neither a significant change nor a change over time in impurities and contents was observed in the amorphous compound.

Thus, it could be seen that the amorphous compound of Example 1 may stably maintain high purity for six months so as to provide excellent stability and a remarkably less occurrence of impurities, thereby maintaining excellent safety for a long period of time with almost no change in moisture content and with almost no absorption of moisture during storage.

### Experimental Example 3. Stress stability test

The solid compound prepared in above Example 1 was left alone under the stress conditions (temperature: 60°C, humidity: 40°C, RH75%) for 0, 7 and 14 days, after which impurities were measured by using high speed liquid chromatography. The results thereof are shown in table 3 below.

**[Table 3]**

| Stress condition | Test item | Example 1 | | |
|---|---|---|---|---|
| | | Zero day (Initial) | 7 days | 14 days |
| Temperature (60°C) | Individual impurity (%) | 0.03 | 0.03 | 0.03 |
| | Total impurity ( % ) | 0.32 | 0.33 | 0.33 |
| | Content (%) | 100.4 | 99.1 | 99.7 |
| Humidity (40°C, RH75%) | Individual impurity (%) | 0.03 | 0.03 | 0.03 |
| | Total impurity ( % ) | 0.32 | 0.32 | 0.32 |
| | Content (%) | 100.4 | 99.5 | 99.9 |

| | | | | |
|---|---|---|---|---|
| (Unit: wt%) | | | | |

From the results of above table 3, neither a significant change nor a change over time in impurities and contents was observed in the amorphous compound.

Thus, it could be seen that the amorphous compound of Example 1 may stably maintain high purity for six months so as to provide excellent stability and a remarkably less occurrence of impurities, thereby maintaining excellent safety for a long period of time with almost no change in moisture content and with almost no absorption of moisture during storage.

### Experimental Example 4. Pharmacokinetic (PK) test

A pharmacokinetic (PK) experiment was performed on the amorphous compound of Example 1.

The powder of the amorphous compound of Example 1 was filled into a capsule in an amount of 100 mg, and the capsule was orally administered to a beagle dog at 100 mg once a day, and then the biokinetics of the drug was confirmed.

Specifically, 100 mg of the amorphous compound was orally administered to the beagle dog, and blood was collected at a predetermined time, so as to measure a concentration of (S)-4-((1-(4,8-dichloro-1-oxo-2-phenyl-1,2-dihydroisoquinoline-3-yl)ethyl)amino)pyrido[2,3-d]pyrimidine-5(8H)-one. With respect to pharmacokinetic parameters, the BA Calc 2007 program was used to calculate an area under the plasma concentration-time curve from dosing time to final plasma concentration quantification time t (AUCₜ), an area under the plasma concentration-time curve from dosing time to infinite time (AUCᵢ), peak plasma concentration (Cₘₐₓ) , time for peak plasma concentration (Tₘₐₓ) , and terminal elimination half-life (t_{1/2}). As a result, a significance between substances was confirmed by Student t-test (SPSS) at a 95% confidence interval, and the results thereof are shown in table 4 below.

**[Table 4]**

| PK Parameter | Amorphous capsule (100mg, PO) |
|---|---|
| t_{1/2}(hr) | 1.81±0.96 |
| Tₘₐₓ (hr) | 1.05±0.68 |
| Cmax (ng/mL) | 450.27±260.00 |
| AUCₗₐₛₜ (ng·hr/mL) | 1152.71±700.01 |
| AUC_{INF} (ng·hr/mL) | 1187.30±716.80 |

As can be confirmed in above table 4, the amorphous form of Example 1 exhibited a high blood concentration.

Thus, it could be seen that the amorphous compound according to Example 1 exhibits excellent bioavailability and thus has a remarkably excellent therapeutic effect.

## Claims

1. An amorphous form of a compound represented by formula 1 below.

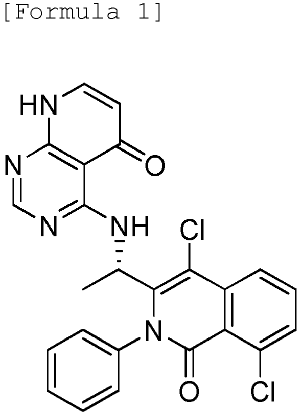

2. The amorphous form according to claim 1, wherein the amorphous compound exhibits a halo pattern in an X-ray powder diffraction pattern.

3. The amorphous form according to claim 1, wherein the amorphous compound has a differential scanning calorimetry (DSC) endothermic transition at 281 to 285°C when a heating rate is 10°C/min.

4. A pharmaceutical composition, comprising the amorphous compound according to any one of claims 1 to 3, and a pharmaceutically acceptable carrier.

5. The pharmaceutical composition according to claim 4, wherein the pharmaceutical composition is used for preventing or treating PIK3-associated diseases.

6. The pharmaceutical composition according to claim 5, wherein the pharmaceutical composition is used for preventing or treating cancer diseases.

7. The pharmaceutical composition according to claim 6, wherein the cancer disease is any one selected from the group consisting of lung cancer, non-small cell lung cancer (NSCL), bronchiolo-alveolar cell lung cancer, gastric cancer, gastrointestinal cancer, liver cancer, bone cancer, pancreatic cancer, skin cancer, head and neck cancer, skin or ocular melanoma, uterine cancer, ovarian cancer, rectal cancer, colorectal cancer, colon cancer, breast cancer, sarcoma of uterus, fallopian tube carcinoma, internal endometrium carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penis cancer, prostate cancer, multiple myeloma, chronic or acute leukemia, solid tumor of childhood, lymphoma, bladder cancer, renal cancer, renal cell carcinoma, renal pelvic carcinoma, spinal axis tumor, brainstem glioma, and pituitary gland adenoma.

8. A method for treating cancer diseases, the method comprising administering a therapeutically effective amount of the amorphous compound according to any one of claims 1 to 3 into a subject.

9. The method according to claim 8, wherein the cancer disease is any one selected from the group consisting of lung cancer, non-small cell lung cancer (NSCL), bronchiolo-alveolar cell lung cancer, gastric cancer, gastrointestinal cancer, liver cancer, bone cancer, pancreatic cancer, skin cancer, head and neck cancer, skin or ocular melanoma, uterine cancer, ovarian cancer, rectal cancer, colorectal cancer, colon cancer, breast cancer, sarcoma of uterus, fallopian tube carcinoma, internal endometrium carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penis cancer, prostate cancer, multiple myeloma, chronic or acute leukemia, solid tumor of childhood, lymphoma, bladder cancer, renal cancer, renal cell carcinoma, renal pelvic carcinoma, spinal axis tumor, brainstem glioma, and pituitary gland adenoma.

10. Use of the amorphous compound according to any one of claims 1 to 3, in the manufacture of a medicament for preventing or treating cancer diseases.

11. The use according to claim 10, wherein the cancer disease is any one selected from the group consisting of lung cancer, non-small cell lung cancer (NSCL), bronchiolo-alveolar cell lung cancer, gastric cancer, gastrointestinal cancer, liver cancer, bone cancer, pancreatic cancer, skin cancer, head and neck cancer, skin or ocular melanoma, uterine cancer, ovarian cancer, rectal cancer, colorectal cancer, colon cancer, breast cancer, sarcoma of uterus, fallopian tube carcinoma, internal endometrium carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penis cancer, prostate cancer, multiple myeloma, chronic or acute leukemia, solid tumor of childhood, lymphoma, bladder cancer, renal cancer, renal cell carcinoma, renal pelvic carcinoma, spinal axis tumor, brainstem glioma, and pituitary gland adenoma.
